# EUROPEAN PATENT SPECIFICATION

(11) **EP 2 741 083 B1**
(45) Date of publication and mention of the grant of the patent: **17.06.2015**
(21) Application number: 13154001.5
(22) Date of filing: 05.02.2013
(51) Int. Cl.: G01N 33/50, B01L 3/00

(54) **Three dimensional microfluidic device that determines metastatic capacity and homing choices**
Dreidimensionale mikrofluidische Vorrichtung, die die metastatische Kapazität bestimmt, und Homing-Auswahlmöglichkeiten
Dispositif microfluidique tridimensionnel qui détermine la capacité métastatique et choix de domiciliation

(30) Priority: 07.12.2012 TR 201214253
(43) Date of publication of application: 11.06.2014
(73) Proprietor: Pesen Okvur, Devrim, 35320 Izmir (TR)
(72) Inventor: Pesen Okvur, Devrim, 35320 Izmir (TR)

(56) References cited:
- WO-A1-2010/018499
- WO-A1-2012/050981
- US-A1- 2012 083 425
- HUIPENG MA ET AL: "Characterization of the interaction between fibroblasts and tumor cells on a microfluidic co-culture device", ELECTROPHORESIS, vol. 31, no. 10, 22 May 2010 (2010-05-22), pages 1599-1605, XP055062929,
- CAROLYN G. CONANT ET AL: "Use of Parallel Flow for Angiogenesis and Cancer Cell Invasion in Microfluidic Devices Using Real-Time Microscopy", BIOPHYSICAL JOURNAL, vol. 100, no. 3, 1 February 2011 (2011-02-01), page 316a, XP055062930,

## Description

### Field of the Invention

The invention concerns a microfluidic device that determines metastatic capacity and homing choices. The device mimics the in vivo tumor microenvironment comprising different cell types, matrices, biological molecules and chemicals. All steps of metastasis, namely, angiogenesis, matrix invasion, cell migration, intravasation, circulation, extravasation and new tumor formation, can be simultaneously and jointly investigated using the said microfluidic device.

### Background of the Invention

The leading cause of death for cancer patients is metastasis. Even if the primary tumor is surgically removed, cells that could have spread from the the primary tumor can cause formation of new tumors and recurrence of cancer. There is no test that can show whether cancer will recur or nor. In addition, as cancer spreads in the body, the tissues where new tumors form can be various. It is not known which type of tissue, cancer cells from each patient will prefer, for new tumor sites. Therefore, it is not possible to determine patient-specific therapies. In short, the desired points in diagnosis and therapy are not reached.

Metastasis of cancerous epithelial cells in the body comprises of steps occuring in a certain order: Cancer cells that have multiplied in an uncontrolled fashion induce angiogenesis, invade tissue matrix (invasion), migrate in tissue matrix towards blood vessels (migration), enter blood vessels (intravasation), move through blood vessels (circulation), exit blood vessels (extravsation) and form tumor in a new tissue. During these molecular events, cancer cells interact with various extracellular molecules (collagen, fibronectin, laminin, growth factors, free radicals, metal ions, etc.) and various cells (macrophages, fibroblasts, endothelial cells, epithelial cells, bone cells, etc.).

Important knowledge on cancer cell biology has been gained due to 2 dimensional and in vitro experiments. However, cells in vivo are in 3D (3 dimensional) matrices. Research shows that cell shape, adhesion, motility, response to growth factors and resistance to drugs are different in two and three dimensional settings¹⁻³. Almost all experiments in vitro and even some animal models can not provide the in vivo orthotopic environment of cancer⁴. Today, only 8 out 100 clinical trials give effective results⁵. 3D cell culture systems have shown that they are a very necessary step between in vitro, in vivo and clinical experiments^{6,7}. Therefore, to achieve a comprehensive understanding of the interactions of cancer with its microenvironment, new cell culture systems are needed.

There are some 3D and some co-culture examples^{8,9}. However,mimicking the in vivo microenvironment is far beyond completion: In vivo, different cell and tissues types exist at certain locations with respect to each other, such as connective tissue being around blood vessels. In addition, cancer metastasis is composed of steps that occur in a certain order. Therefore, a system that will investigate these steps has to mimic the in vivo microenvironment.

Needle biopsy is used to predict metastatic capacity. It is also proposed that gene signatures will be useful¹⁰. However, functional tests at the cell and/or tissue levels do not exist. Needle biopsy is a structural test because it determines the organization of cells taken from the patient checking whether cells are connected to eacah other or are dispersed. On the other hand, a functional test would check whether cells carry out the steps of metastasis: angiogenesis, matrix invasion, cell migration, intravasation, circulation, extravasation and new tumor formation, or not.

Microfluidic technology provides precise spatial and temporal control, high-throughput analysis, low fabrication costs ve portability. Used material and waste volumes can be as low as picoliters. Using small volumes of unknown or toxic materials provides safe experimental study. Moreover, microfluidic technology can provide means to mimic physiological microenvironments. This feature can help us more realistically study cells in both health and disease states and improve drug testing approches. It can also help reduce animal testing.

With microfluidic technology based set-ups, some steps of cancer cell metastasis have been studied to certain degree. Research on breast cancer cells and fibroblast cells together in 3D collagen¹¹, angiogenesis due to growth factors in microfluidic channels and interactions of cancer cells with endothelial cells^{12,13}, effects of drugs in microfluidic channels on cancer cells in 3D cell culture¹⁴, hepatocyte cell culture in microfluidic systems¹⁵, interactions of breast cancer cells and macrophages in 3D microfluidic channels¹⁶, interactions of breast cancer cells and fibroblasts in 3D microfluidic channels¹⁷, interactions of breast cancer cells, macrophages and endothelial cells in 3D microfluidic channels¹⁸ have been perfomed. However, there is neither a set-up nor a device that can simultaneously and jointly investigate all steps of cancer cell metastasis and that can mimic the required features of the in vivo microenvironment.

The patent which has the closest content to the submitted application here is the patent by Roger Kamm, titled "Device for High Throughput Investigations of Cellular Interactions" ¹⁹. The basic unit in the mentioned patent, is composed of various flow channels around one microfluidic channel/area comprising 3D cell-laden matrix. By repeating the basic unit high-throughput is achieved. Since there is only one microfluidic channel/area comprising 3D cell-laden matrix in each basic unit, it is not possible for different cell types to be studied to be in neighboring but separate microfluidic channels/areas comprising 3D cell-laden matrices. Thus, cells to be studied together are mixed in 3D matrix and loaded into the microfluidic channel/area comprising 3D cell-laden matrix. In vivo, there can be more than one type of cell in one region, for example both macrophages and fibroblasts can be found in connective tissue; however different cell types are ofund in different tissues and organs: For example lung epithelial cells are found in lungs, breast epithelial cells are found in breast. For example in breast cancer, cancer epithelial cells are next to normal epithelial cells when they first form a tumor. When cancer epithelial cells pass into connective tissue, they come next to macrophages and fibroblasts that can be found together in connective tissue. As cancer epithelial cells spread in the body, they interact with different cell types such as endothelial cells, lung epithelial cells, live epithelial cells, bone cells found in different regions in the body. To be able to study cancer metastasis outside the organism, it is necessary to mimic the in vivo organization of different cell types in different regions, outside the organism. Therefore, the device in Kamm's patent, due to having only one 3D cell-laden matrix channel/area in its basic unit, is limited in mimicking the in vivo microenvironment. Hence, at most three different cell types could have been simultaneously studied. In addition, the mentioned device does not make it possible to investigate all steps of cancer metastasis simultaneously and jointly. Another deficiency is that the device cannot determine homing choices of cancer cells. For example, it cannot determine whether breast cancer cells that have entered blood flow will form new tumors in regions with 3D lung epithelial cell-laden matrix or 3D liver epithelial cell-laden matrix or 3D bone cell-laden matrix.

On the other hand, the device I present in this patent application, due to its basic unit comprising at least one flow channel and at least 3 on each side, at least 6 in total, channels, comprising 3D cell-free or cell-laden matrices neighbouring each flow channel, achieves (i) simultaneous investigation of at least five different cell types, (ii) simultaneous investigation of all steps of cancer metastasis and thus determination of metastatic capacity, and (iii) determination of homing choices of cancer cells.

To determine metastatic capacity, all steps of metastasis should be investigated simultaneously and jointly because each step is connected to others. Cancer cells less successful at one or more steps can still metastasize. The important question is whether new tumors form or not as a result of the sum of all steps. This is the question the drugs that will be used against cancer are expected to answer. More important than which metastatic step the drug affects is whether the drug prevents new tumor formation or not. Side effects of anti-cancer drugs on normal cells is an unwanted situation. It is ideal to use cell culture devices comprising 3d matrices, multiple and different cell types and thus best mimicking the in vivo conditions, to investigate side effects.

### Summary of the invention

The purpose of the invention is to determine metastatic capacity of cancer cells.
Another purpose of the invention is to determine homing choices of cancer cells.
Another purpose of the invention is to simultaneously and jointly investigate all steps of metastasis, namely, angiogenesis, matrix invasion, cell migration, intravasation, circulation, extravasation and new tumor formation under conditions mimicking the in vivo microenvironment.
Another purpose of the invention is to test anti-metastasis drugs.

The microfluidic device with the desccribed features is shown in the drawings below.
The drawings are not necessarily to scale.

### Brief description of the drawings

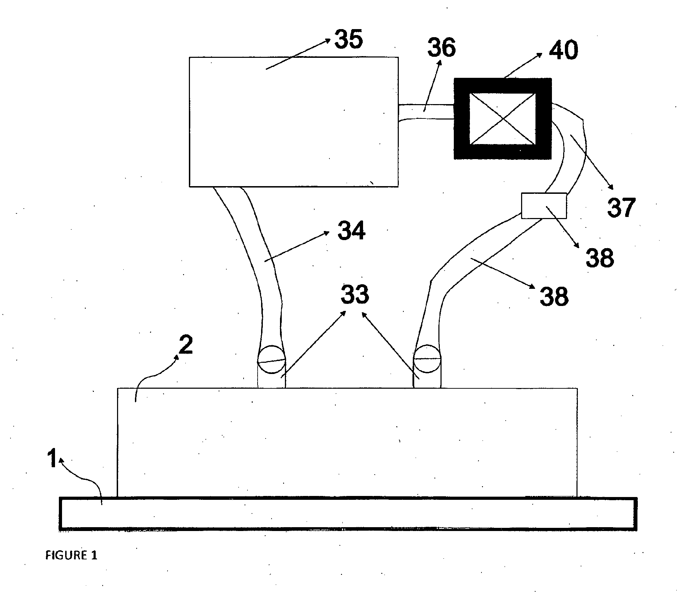

Fig. 1 shows a drawing of a longitudinal outside view of a device and presentation of peripheral units

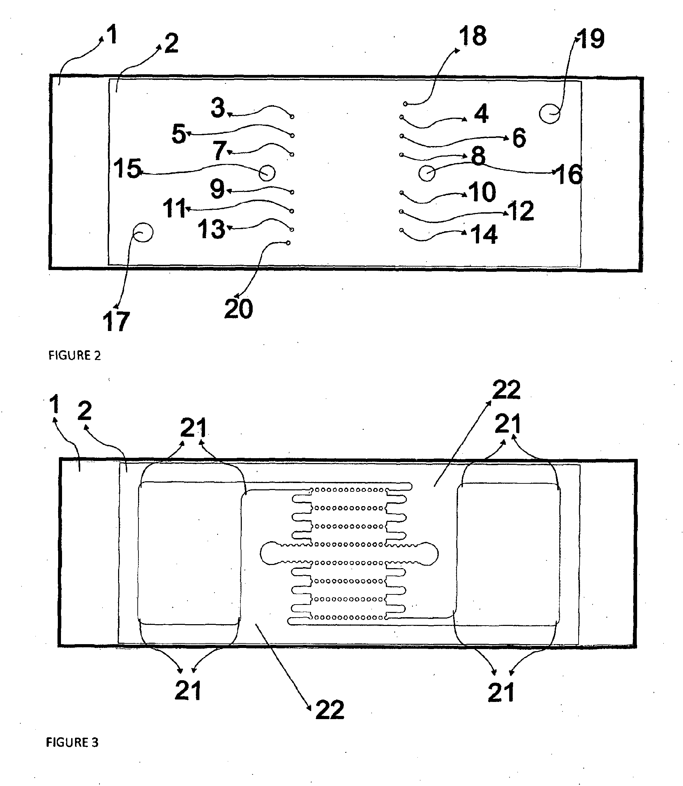

Fig. 2 shows a drawing of a longitudinal, top outside section view of a device
Fig. 3 shows a drawing of a longitudinal, top inside section view of a device with references 21 and 22

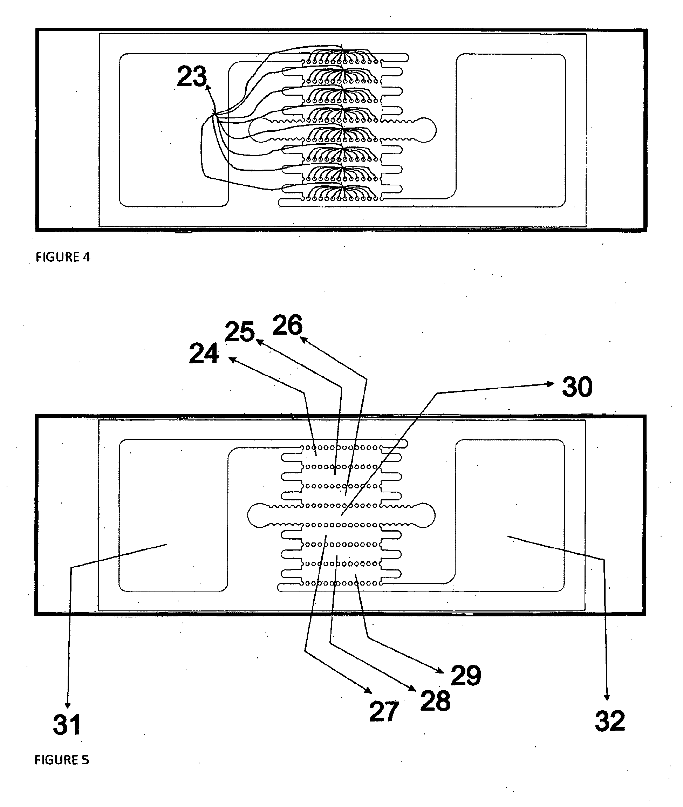

Fig. 4 shows a drawing of a longitudinal, top inside section view of a device with references 23
Fig. 5 shows a drawing of a longitudinal, top inside section view of a device with references 24, 25, 26, 27, 28, 29, 30, 31 and 32

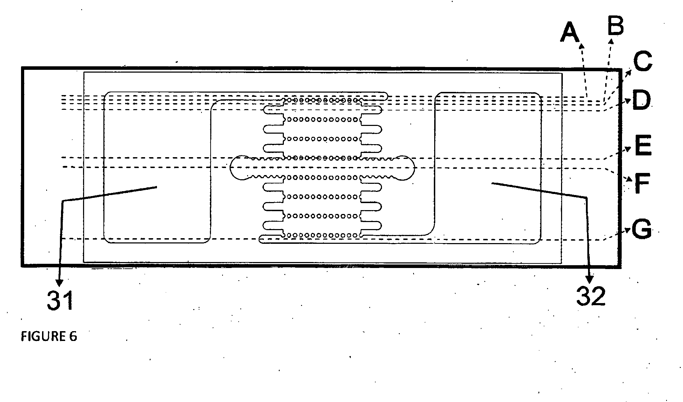

Fig. 6 shows a drawing of the places of cross-sections at the longitudinal, top inside section view of a device

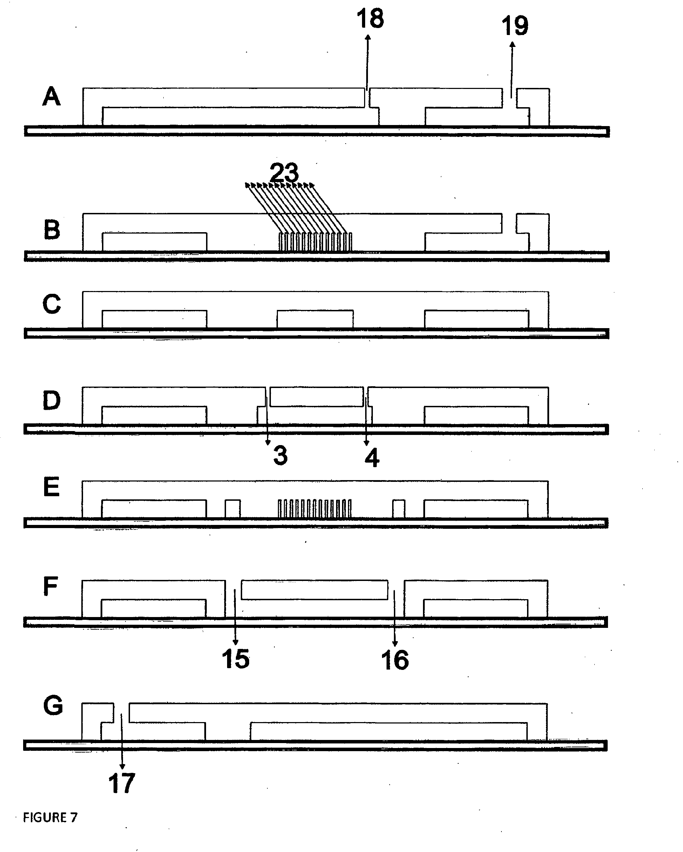

Fig. 7 shows a drawing of the cross-sections taken at places noted in Fig. 6

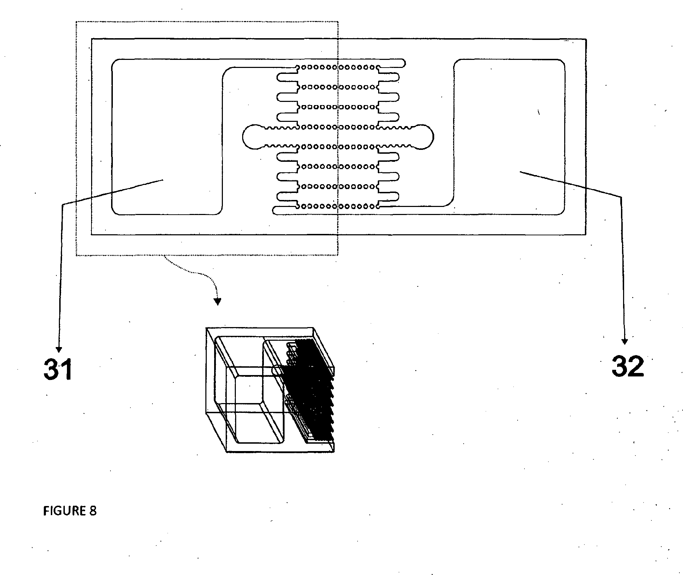

Fig. 8 shows a drawing of a longitudinal, top inside section view of a device and the 3D view of selected region of the device including a fluid reservoir

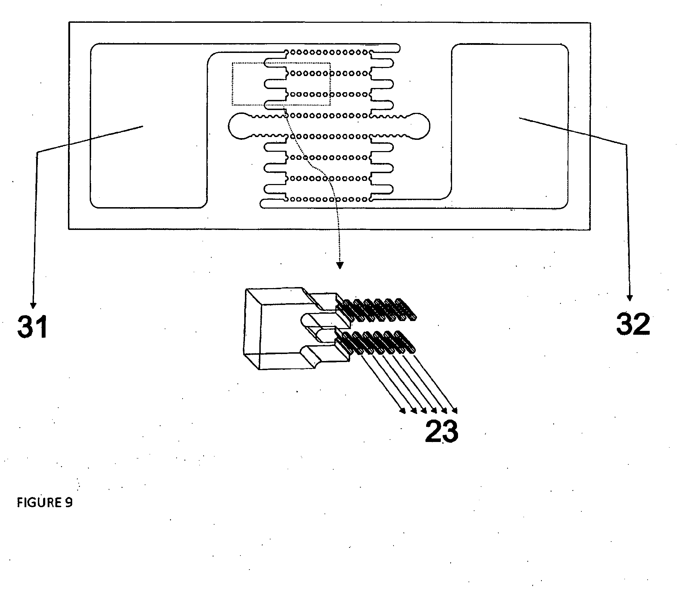

Fig. 9 shows a drawing of a longitudinal, top inside section view of a device and the 3D view of selected region of the device including two partial rows of posts.

### Brief description of the references in the drawings

Parts in the figures are numbered and their explanations are given below:
1: The optically transparent surface that forms the base of the device
2: The structure of the device
3: Channel inlet or outlet
4: Channel inlet or outlet
5: Channel inlet or outlet
6: Channel inlet or outlet
7: Channel inlet or outlet
8: Channel inlet or outlet
9: Channel inlet or outlet
10: Channel inlet or outlet
11: Channel inlet or outlet
12: Channel inlet or outlet
13: Channel inlet or outlet
14: Channel inlet or outlet
15: Channel inlet or outlet
16: Channel inlet or outlet
17: Fluid reservoir inlet
18: Fluid reservoir outlet
19: Fluid reservoir inlet
20: Fluid reservoir outlet
21: Curved interior corner
22: Border area that prevents direct mixing of fluids in fluid reservoirs
23: Post
24: 3D cell-free or cell-laden matrix channel
25: 3D cell-free or cell-laden matrix channel
26: 3D cell-free or cell-laden matrix channel
27: 3D cell-free or cell-laden matrix channel
28: 3D cell-free or cell-laden matrix channel
29: 3D cell-free or cell-laden matrix channel
30: Flow channel
31: Fluid reservoir
32: Fluid reservoir
33: Connector
34: Tubing
35: Storage reservoir
36: Tubing
37: Tubing
38: Air bubble trap
39: Tubing
40: Flow regulating pump

### Detailed description of the invention

The invention is a microfluidic device that can be used to simultaneously and jointly investigate all steps of metastasis and homing choices of cancer cells. The device mimics the in vivo tissue level organization of tumor microenvironments comprising various tissues and blood vessels. Different tissues are mimicked by at least six different channels comprising cell-laden or cell-free 3D matrices (24, 25, 26, 27, 28, 29). Blood vessels are mimicked by at least one flow channel (30) comprising endothelial cells. Channels comprising cell-laden or cell-free 3D matrices (24, 25, 26, 27, 28, 29) are arranged along each long side of the flow channel (30) and one of the channels on one side of the flow channel (30) comprises cancer cell laden matrix and all others comprise cell free or normal cell laden matrices.

In one embodiment, the structure (2) on the optically transparent surface has the following parts: at least one flow channel (30) comprising endothelial cells on its walls, at least three adjacent channels comprising cell-laden or cell-free 3D matrices, neighboring each flow channel (30) on each long side of it, with same or different lengths as the flow channel (30), in total at least six channels comprising cell-laden or cell-free 3D matrices (24, 25, 26, 27, 28, 29), at least two fluid reservoirs (31, 32) each neighbouring one of the channels comprising cell-laden or cell-free 3D matrices (24, 29) furthest from the flow channel (30), at least two border areas that prevent direct mixing of different fluids in the fluid reservoirs (22), borders comprising at least twelve posts (23) that separate neighboring channels (24, 25, 26, 27, 28, 29, 30) from each other, and channels (24, 29) from neighboring fluid reservoirs (31, 32), curved interior corners (21) that minimize air bubble formation.

There are at least three 3D cell-free or cell-laden matrix comprising channels adjacent to eac other and to each side of each flow channel (30). Cells and/or molecules from at least six 3D cell-free or cell-laden matrix comprising channels can reach each flow channel through spacings between posts. If the number of flow channels (30) is increased, number of 3D cell-free or cell-laden matrix containing channels will increase accordingly. If there are 2 flow channels (30), there will be at least 12 of 3D cell-free or cell-laden matrix containing channels.

Fluid reservoirs (31, 32) provide biological molecules and chemicals to cells furthest away from the flow and mimic interstitial fluid.

One end of the flow channel (30) is connected via connector (33) and tubing (39) to air bubble trap (38) connected via tubing (37) to flow regulating pump (40) and the other end of the flow channel is connected via connector (33) and tubing (34) to a storage reservoir (35) where flow passes through and cancer cells and other biological molecules and chemicals that have entered flow can be collected for analysis. The storage reservoir (35) is connected via tubing (36) to the flow regulating pump (40).

All channels (24, 25, 26, 27, 28, 29, 30) and fluid reservoirs (31,32) in the device have each one inlet for loading culture media, physiological buffer solution, biological molecules or chemicals to be tested, cell-free matrix, cell-laden matrix or a combination thereof and each one outlet for inside air or preloaded fluid to exit during loading (3, 4, 5, 6, 7, 8, 9, 10, 11, 12, 13, 14, 15, 16, 17, 18, 19, 20).

If the number of flow channels (30) is increased, numbers of inlets, outlets (15, 16), connectors (33), inlet and outlet tubings (34, 39), and if desired number of storage reservoirs (35), inlet and outlet tubings (34, 36) of storage reservoirs will be increased accordingly.

If the border areas that prevent direct mixing of fluids in the fluid reservoirs (22) are not used, the fluids in the two fluid reservoirs can have the same composition.

The fact that the interior corners are curved can reduce air bubbles that can form inside the device during fluid loading.

All channels (24, 25, 26, 27, 28, 29, 30) in the device are separated from each with rows of posts (23). Fluid reservoirs (31, 32) are also separated from the outermost channels (24, 29) with rows of posts (23). There are at least 12 posts (23) in each row. Since the separation of channels and fluid reservoirs is realized with rows of posts instead of solid walls, cells, biological molecules and/or chemicals in one channel and/or reservoir can pass to other channel and/or reservoirs.

All channels (24, 25, 26, 27, 28, 29, 30), fluid reservoirs (31,32) and posts (23) are of the same height. Their height can be between 50 micrometers and 5 millimeters.
Width and length of each channel (24, 25, 26, 27, 28, 29,30) can be the same as or different from the widths and lengths of other channels. Widths of channels can be between 100 micrometers and 25 milimeters.
The horizontal cross-section of each post (23) can have the shape of a hexagon, a circle or an ellipse. The width of each post can be between 50 micrometers to 3 millimeters.
The spacing between two consecutive posts (23) is shorter than the width of the post (23) which has the smaller width of the two consecutive posts (23).
The diameters of inlet and outlets in the device (3, 4, 5, 6, 7, 8, 9, 10, 11, 12, 13, 14, 15, 16, 17, 18, 19, 20) can be between 50 micrometers and 10 milimeters.

When more than one device is used, the devices can have separate optically transparent surfaces (1) or their structures (2) can be organized on one common optically transparent surface.

The material of the optically transparent surface (1) can be glass, polydimethylsiloxane (PDMS) or polystyrene (PS).

The material of the structure (2) on the optically transparent surface (1) can be polydimethylsiloxane (PDMS) or polystyrene.

The structure (2) on the optically transparent surface (1) can be fabricated by polymerizing PDMS on silicon or SU-8 masters prepared with standard lithography techniques, or by polymerizing PS on PDMS masters prepared with standard lithography techniques, or by injection molding PS. The optically transparent surface (1) and the structure can be bonded using UV/ozone treatment, plasma treatment and/or heating.

Cell lines and/or cancer patient biopsy cells such as cancer cells, macrophages, fibroblasts, endothelial cells, normal breast epithelial cells, myoepithelial cells, normal liver epithelial cells, hepatocytes, normal lung epithelial cells, normal bone cells can be used in the device. At least five different cell types can be simultaneously and jointly investigated in the device. Normal epithelial cells of type same as the cancer type or different from the cancer type can be used. For example, if breast cancer cells will be studied, breast cancer epithelial cells and normal breast epithelial cells and/or normal lung epithelial cells can be used.
Example 1: 1. Breast cancer epithelial cells 2. normal breast epithelial cells 3. fibroblasts 4. macrophages 5. endothelial cells.
Example 2: 1. Breast cancer epithelial cells 2. normal breast epithelial cells 3. fibroblasts 4. macrophages 5. endothelial cells 6. bone cells
Example 3: 1. Breast cancer epithelial cells 2. normal breast epithelial cells 3. fibroblasts 4. macrophages 5. endothelial cells 6. liver cells
Example 4: 1. Breast cancer epithelial cells 2. normal breast epithelial cells 3. fibroblasts 4. macrophages 5. endothelial cells 6. normal lung epithelial cells
Example 5: 1. Breast cancer epithelial cells 2. normal breast epithelial cells 3. fibroblasts 4. macrophages 5. endothelial cells 6. normal lung epithelial cells 7. bone cells
A sample placement of cells in the device for the Example 5 above: Breast cancer epithelial cells in matrix in relevant channel (24), normal breast epithelial cells in matrix in relevant channel (25), fibroblasts and macrophages in matrix in relevant channel (26), endothelial cells in flow channel (30), fibroblasts and macrophages in matrix in relevant channel (27),normal lung epithelial cells in matrix in relevant channel (28), bone cells in matrix in relevant channel (29).

Blood of cancer patient can be used as fluid passing through the flow channel (30). In this case, metastatic capacity of cancer cells that have already entered blood circulation can be investigated. Blood of healthy individual can be used as control.

A sample application is as follows:
Cell-free matrix is loaded into relevant channels (24, 29),
Cancer cell laden matrix is loaded into relevant channel (25),
Macrophage and fibroblast laden matrix is loaded into relevant channels (26, 27),
Normal epithelial cell laden matrix is loaded into relevant channel (28).
After matrices polymerize, endothelial cells are loaded into the flow channel (30) and culture media are loaded into fluid reservoirs (31, 32).
The storage reservoir (35) is filled with physiological buffer.
The flow regulating pump (40), air bubble trap (38), storage reservoir (35) and tubings (34, 36, 37, 39) are connected to each other.
The flow channel (30) is connected to flow via connectors (33) and tubings (34, 39). Tubings (34, 39) can be directly placed in inlet and outlet (15, 16) without using connectors (33) if desired.
Device is placed on microscope stage. Device is kept at 37°C. Device is also kept at 5% CO₂ atmosphere unless CO₂ independent culture media are used.
After endothelial cells form a monolayer, flow is started. Microscope images are taken at regular time intervals at predefined positions in the device. Cell behavior is observed, recorded and analyzed. Or at a predetermined time point, for example 3 days, 7 days, 14 days, cells in the device are labeled using standard immunohistochemistry to determine locations of cancer cells and other normal cells of interest, for example, endothelial cells, macrophages, normal epithelial cells, etc.
If cancer cells reach normal epithelial cell laden matrix comprising channel (28) and form new tumors, they are classified as cancer cells with high metastatic capacity.

The device can be used to determine whether a biological molecule or a chemical or a combination thereof prevents metastasis or not. A sample application is as follows:
Cancer cell laden matrix is loaded into relevant channel (24),
Fibroblast laden matrix is loaded into relevant channels (25, 28),
Macrophage laden matrix is loaded into relevant channels (26, 27),
Normal epithelial cell laden matrix is loaded into relevant channel (29).
After matrices polymerize, endothelial cells are loaded into the flow channel (30) and culture media are loaded into fluid reservoirs (31, 32).
The storage reservoir (35) is filled with physiological buffer and biological molecule, chemical or a combination thereof to be tested.
The flow regulating pump (40), air bubble trap (38), storage reservoir (35) and tubings (34, 36, 37, 39) are connected to each other.
The flow channel (30) is connected to flow via connectors (33) and tubings (34, 39). Tubings (34, 39) can be directly placed in inlet and outlet (15, 16) without using connectors (33) if desired.
Device is placed on microscope stage. Device is kept at 37°C. Device is also kept at 5% CO₂ atmosphere unless CO₂ independent culture media are used.
After endothelial cells form a monolayer, flow is started. Microscope images are taken at regular time intervals at predefined positions in the device. Cell behavior is observed, recorded and analyzed. Or at a predetermined time point, for example 3 days, 7 days, 14 days, cells in the device are labeled using standard immunohistochemistry to determine locations of cancer cells and other normal cells of interest, for example, endothelial cells, macrophages, normal epithelial cells, etc.
If new tumors form in the normal epithelial cell laden matrix (29) comprising channel when controls of biological molecule, chemical or a combination thereof is used and new tumors do not form in the normal epithelial cell laden matrix comprising channel (29) when the biological molecule, or chemical or a combination thereof tested is used, then this indicates the biological molecule, or chemical or a combination thereof tested can be used against cancer metastasis.

The device can be used to determine the homing choices of cancer cells. For example to determine whether breast cancer cells will metastasize to lungs or bones:
in one device breast cancer cell-laden matrix can be loaded into one channel on one side of the flow channel (30) and normal bone cell-laden matrix into another channel on the other side of the flow channel (30) and in another device breast cancer cell-laden matrix can be loaded into one channel on one side of the flow channel (30) and normal lung epithelial cell-laden matrix into another channel on the other side of the flow channel (30) or
in one device breast cancer cell-laden matrix can be loaded into one channel on one side of the flow channel (30) and normal lung epithelial cell-laden matrix into one half of another channel and normal bone cell-laden matrix into the other half of the channel on the other side of the flow channel (30) or in one device breast cancer cell-laden matrix can be loaded into one channel on one side of the flow channel (30), normal lung epithelial cell-laden matrix and normal bone cell-laden matrix into other channels on the other side of the flow channel (30).
Then where breast cancer cells migrate and where they form new tumors in each device can be determined.

For example to determine whether breast cancer cells will metastasize to lungs or bones an application is as follows:
Normal epithelial cell laden matrix is loaded into relevant channel (24),
Cancer cell laden matrix is loaded into relevant channel (25),
Macrophage and fibroblast laden matrix is loaded into relevant channels (26, 27),
Normal lung epithelial cell laden matrix is loaded into relevant channel (28).
Normal bone cell laden matrix is loaded into relevant channel (29).
After matrices polymerize, endothelial cells are loaded into the flow channel (30) and culture media are loaded into fluid reservoirs (31, 32).
The storage reservoir (35) is filled with physiological buffer.
The flow regulating pump (40), air bubble trap (38), storage reservoir (35) and tubings (34, 36, 37, 39) are connected to each other.
The flow channel (30) is connected to flow via connectors (33) and tubings (34, 39). Tubings (34, 39) can be directly placed in inlet and outlet (15, 16) without using connectors (33) if desired.
Device is placed on microscope stage. Device is kept at 37°C. Device is also kept at 5% CO₂ atmosphere unless CO₂ independent culture media are used.
After endothelial cells form a monolayer, flow is started. Microscope images are taken at regular time intervals at predefined positions in the device. Cell behavior is observed, recorded and analyzed. Or at a predetermined time point, for example 3 days, 7 days, 14 days, cells in the device are labeled using standard immunohistochemistry to determine locations of cancer cells and other normal cells of interest, for example, endothelial cells, macrophages, normal epithelial cells, etc.
If cancer cells reach normal lung epithelial cell laden matrix comprising channel (28) and form new tumors, they are classified as cancer cells that home to lung tissue. If cancer cells reach normal bone cell laden matrix comprising channel (29) and form new tumors, they are classified as cancer cells that home to bone tissue.

To distinguish cancer cells from other cells, they can be labelled with fluorescent dyes before loading into the device. Another approach is to label cells other than the cancer cells to be loaded into the device with fluorescent dyes. Or these cells can express various fluorescent proteins.

Effect of different flow rate on cancer cell metastasis can be invesitgated by changing the flow rate generated with the flow regulating pump (40).

The matrix can be collagen, matrigel, laminin, hydrogel or a combination thereof. In addition, extracellular matrix proteins such as fibronectin, entactin can be added to the matrix.

Drugs, growth factors, biological molecules and/or chemicals to be tested can be added to one or more of the reservoirs (31, 32, 35). Intended effects on cancer cells and side effects on normal cells can be simultaneously and jointly studied.

When cells from cancer patient biopsies are used in the device, better informed choices can be made for personalized therapy.

The device can be fabricated using standard lithography, hot embossing, micro-injection molding and/or laser micromachining techniques.

Loading of materials to the device, change of fluids, microscopic observations and analyses can be automized with a computerized robotic set-up.

### REFERENCES

1 Cukierman, E., Pankov, R., Stevens, D. R. & Yamada, K. M. Taking cell-matrix adhesions to the third dimension. Science 294, 1708-1712 (2001).
2 Faute, M. A. D. et al. Distinctive alterations of invasiveness, drug resistance and cell-cell organization in 3D-cultures of MCF-7, a human breast cancer cell line, and its multidrug resistant variant. Clinical & Experimental Metastasis 19, 161-168 (2002).
3 Kim, H. D. et al. Epidermal Growth Factor-induced Enhancement of Glioblastoma Cell Migration in 3D Arises from an Intrinsic Increase in Speed But an Extrinsic Matrix- and Proteolysis-dependent Increase in Persistence. Mol Biol Cell 19, 4249-4259, doi:10.1091/mbc.E08-05-0501 (2008).
4 McMillin, D. W. et al. Tumor cell-specific bioluminescence platform to identify stroma-induced changes to anticancer drug activity. Nature Medicine 16, 483-U171, doi:10.1038/nm.2112 (2010).
5 Woodcock, J. & Woosley, R. The FDA critical path initiative and its influence on new drug development. Annual Review of Medicine 59, 1-12, doi:10.1146/annurev.med.59.090506.155819 (2008).
6 Debnath, J. & Brugge, J. S. Modelling glandular epithelial cancers in three-dimensional cultures. Nature Reviews Cancer 5, 675-688, doi:10.1038/nrc1695 (2005).
7 Hutmacher, D. W. et al. Can tissue engineering concepts advance tumor biology research? Trends in Biotechnology 28, 125-133, doi:10.1016/j.tibtech.2009.12.001 (2010).
8 Olumi, A. F. et al. Carcinoma-associated fibroblasts direct tumor progression of initiated human prostatic epithelium. Cancer Res 59, 5002-5011 (1999).
9 Weaver, V. M. et al. Reversion of the malignant phenotype of human breast cells in three-dimensional culture and in vivo by integrin blocking antibodies. J Cell Biol 137, 231-245 (1997).
10 van't Veer, L. J. et al. Gene expression profiling predicts clinical outcome of breast cancer. Nature 415, 530-536 (2002).
11 Bauer, M., Su, G., Beebe, D. J. & Friedl, A. 3D microchannel co-culture: method and biological validation. Integr Biol-Uk 2, 371-378 (2010).
12 Chung, S., Sudo, R., Vickerman, V., Zervantonakis, I. K. & Kamm, R. D. Microfluidic Platforms for Studies of Angiogenesis, Cell Migration, and Cell-Cell Interactions. Ann Biomed Eng 38, 1164-1177 (2010).
13 Shin, Y. et al. Microfluidic assay for simultaneous culture of multiple cell types on surfaces or within hydrogels. Nat Protoc 7, 1247-1259 (2012).
14 Elliott, N. T. & Yuan, F. A microfluidic system for investigation of extravascular transport and cellular uptake of drugs in tumors. Biotechnol Bioeng 109, 1326-1335 (2012).
15 Goral, V. N. et al. Perfusion-based microfluidic device for three-dimensional dynamic primary human hepatocyte cell culture in the absence of biological or synthetic matrices or coagulants. Lab Chip 10, 3380-3386 (2010).
16 Huang, C. P. et al. Engineering microscale cellular niches for three-dimensional multicellular co-cultures. Lab Chip 9, 1740-1748 (2009).
17 Sung, K. E. et al. Transition to invasion in breast cancer: a microfluidic in vitro model enables examination of spatial and temporal effects. Integr Biol-Uk 3, 439-450 (2011).
18 Zervantonakis, I. K. et al. Three-dimensional microfluidic model for tumor cell intravasation and endothelial barrier function. P Natl Acad Sci USA 109, 13515-13520 (2012).
19 Kamm, R. D. *et al.* Device for High Throughput Investigations of Cellular Interactions. PCT/US2011/054029 (2011).

## Claims

1. A microfluidic device comprising a structure (2) on an optically transparent surface (1) where the structure (2) comprises
(i) at least one flow channel (30) comprising endothelial cells,
(ii) at least three adjacent channels comprising cell-laden or cell-free 3D (3 dimensional) matrices, neighboring each flow channel on each long side of it, in total at least six channels comprising 3D cell-laden or cell free matrix (24, 25, 26, 27, 28, 29),
(iii) at least two fluid reservoirs (31, 32) each neighbouring one of the channels comprising 3D cell-laden or cell free matrix (24, 29) furthest from the flow channel (30),
(iv) borders comprising at least twelve posts (23) that separate neighboring channels (24, 25, 26, 27, 28, 29, 30) from each other, and channels (24, 29) from neighboring fluid reservoirs (31, 32).
(v) at least five different cell types in total.

2. The device of claim 1 wherein one end of the flow channel (30) is connected via connector (33) and tubing (39) to air bubble trap (38) connected to a flow regulating pump (40) and the other end is connected via connector (33) and tubing (34) to a storage reservoir (35) where the flow passes through and cancer cells, biological molecules and chemicals that have entered the flow can be collected for analysis.

3. The device of claim 1 wherein there are at least two border areas (22) that prevent mixing of different fluids in the fluid reservoirs (31, 32),

4. The device of claim 1 wherein the interior corners (21) are curved to minimize air bubble formation.

5. The device of claim 1 wherein the fluids in the reservoirs (31, 32, 35) and the flow channel (30) comprise culture media, physiological buffer solutions, biological molecules, chemicals to be tested or a combination thereof.

6. The device of claim 1 wherein all channels (24, 25, 26, 27, 28, 29, 30) and fluid reservoirs (31,32) comprise each one inlet for loading culture media, physiological buffer solutions, biological molecules, chemicals, cell-free matrix, cell-laden matrix to be tested or a combination thereof and each one outlet for the inside air or preloaded fluid to exit during loading (3, 4, 5, 6, 7, 8, 9, 10, 11, 12, 13, 14, 15, 16, 17, 18, 19, 20).

7. The device of claim 1 wherein channels (24, 25, 26, 27, 28, 29, 30), fluid reservoirs (31,32) and posts (23) are of the same height.

8. The device of claim 1 wherein channels comprising 3D cell-laden or cell free matrix (24, 25, 26, 27, 28, 29) are the same or different lengths as the flow channel (30),

9. The device of claim 1 wherein all channels (24, 25, 26, 27, 28, 29, 30) are parallel to each other.

10. The device of claim 1 wherein all channels (24, 25, 26, 27, 28, 29, 30) each have a length between 500 micrometers and 20 centimeters.

11. The device of claim 1 wherein the horizontal cross-section of each post (23) is hexagonal, circular or elliptical.

12. The device of claim 1 wherein the horizontal cross-section of each post (23) is between 10 micrometers and 1 millimeter wide.

13. The device of claim 1 wherein the material of the optically transparent surface(1) is glass, polydimethylsiloxane or polystyrene.

14. The device of claim 1 wherein the material of the structure (2) is polydimethylsiloxane or polystyrene.

15. The device of claim 1 wherein the cells comprise a combination of cancer cells, macrophages, fibroblasts, endothelial cells, normal breast epithelial cells, myoepithelial cells, normal liver epithelial cells, hepatocytes, normal lung epithelial cells, normal bone cells.

16. The device of claim 15 wherein the cells are cell lines and/or cancer patient biopsy cells.

17. The device of claim 1 wherein at least one of the channels (24, 25, 26, 27, 28, 29) comprising 3D cell-laden or cell free matrix, comprises 3D cancer cell-laden matrix while the others comprise 3D cell-free matrix, 3D normal epithelial cell of type same as the cancer type-laden matrix, 3D normal epithelial cell of type different from the cancer type-laden matrix, 3D fibroblast-laden matrix, 3D macrophage-laden matrix, 3D myoepithelial cell-laden matrix, 3D normal liver epithelial cell-laden matrix, 3D hepatocyte-laden matrix, 3D normal bone cell-laden matrix or a combination thereof.

18. The device of claim 1 wherein the 3D matrix in six or fewer of the channels (24, 25, 26, 27, 28, 29) comprising 3D cell-laden or cell free matrix is matrigel, collagen, laminin or a combination thereof.

19. The device of claim 1 wherein the flow channel (30) is a channel where
(i) Cancer cells that contact endothelial cells in the flow channel (30),
(ii) Cancer cells that pass endothelial cells in the flow channel (30) and enter the flow,
(iii) Cancer cells that enter the flow on one side of the flow channel and contact the endothelial cells on the other side of the flow channel (30) are observed with a microscope.

20. The device of claim 1 wherein the structure comprises channels (26, 27) neighboring the flow channel (30) and comprising 3D cell-laden or cell free matrix where migration of cancer cells in 3D cell-laden or cell free matrix towards the flow channel (30) and exit of cancer cells from the flow channel (30) into 3D cell-laden or cell free matrix can be observed.

21. The device of claim 1 wherein any of the channels (24, 25, 26, 27, 28, 29) comprising 3D normal cell of type same as the cancer type or different type-laden matrix is a channel where formation and/or presence of new tumors by cancer cells that have passed through the flow channel (30) and/or not, can be observed with a microscope.

22. The device of claim 1 wherein the fluid in the flow channel (30) is blood from cancer patient.

23. The device of claim 1 wherein the fluid in the flow channel (30) is blood from healthy individual.

24. A method of determining whether a biological molecule or a chemical or a combination thereof prevents metastasis or not comprising
a. adding the biological molecule, chemical or a combination thereof to be tested into one or more of the fluid reservoirs (31, 32) and/or storage reservoir (35) of the device in claim 1 wherein at least one of the channels (24, 25, 26, 27, 28, 29) comprising 3D cell-laden or cell free matrix comprises 3D cancer cell-laden matrix while the others comprise 3D cell-free matrix, 3D normal epithelial cell of type same as the cancer type-laden matrix, 3D normal epithelial cell of type different from the cancer type-laden matrix, 3D fibroblast-laden matrix, 3D macrophage-laden matrix, 3D myoepithelial cell-laden matrix, 3D normal liver epithelial cell-laden matrix, 3D hepatocyte-laden matrix, 3D normal bone cell-laden matrix or a combination thereof, and
b. determining whether new tumors form in a channel other than the one seeded with cancer cells to begin with or not
wherein if new tumors do not form in a channel other than the one seeded with cancer cells to begin with, then this indicates that the biological molecule, chemical or a combination thereof tested can be used against metastasis compared to a suitable control biological molecule or chemical.

25. A method of determining the homing choices of cancer cells comprising
a. The device of claim 1 wherein at least one of the channels (24, 25, 26, 27, 28, 29) comprising 3D cell-laden or cell free matrix comprises 3D cancer cells to be tested-laden matrix while the others comprise 3D cell-free matrix, 3D normal epithelial cell of type same as the cancer type-laden matrix, 3D normal epithelial cell of type different from the cancer type-laden matrix, 3D fibroblast-laden matrix, 3D macrophage-laden matrix, 3D myoepithelial cell-laden matrix, 3D normal liver epithelial cell-laden matrix, 3D hepatocyte-laden matrix, 3D normal bone cell-laden matrix or a combination thereof, and
b. determining in which channels new tumors form or are present
wherein if a new tumor forms in a channel with normal lung epithelial cells, then this indicates that cancer cells tested will metastasize to the lungs, if a new tumor forms in a channel with bone cells, then this indicates that cancer cells tested will metastasize to the bones, if a new tumor forms in a channel with normal liver cells, then this indicates that cancer cells tested will metastasize to the liver.

## Patentansprüche

1. Die Erfindung richtet sich auf eine mikrofluidische Vorrichtung, **dadurch gekennzeichnet, dass** das auf der lichtdurchlässigen Oberfläche (1) befindliche Gewebe (2) der Vorrichtung aus
(i) mindestens einem Durchflusskanal (30), der die Endothelzellen enthält,
(ii) mindestens drei benachbarten Kanälen, die an jede lange Seite eines jeden Durchflusskanals angrenzen und eine 3-D (3-dimensionale)-Matrix mit oder ohne Zellen enthalten; insgesamt mindestens sechs Matrixkanälen (24, 25, 26, 27, 28, 29), die eine 3-D-Matrix mit oder ohne Zellen enthalten,
(iii)mindestens zwei Flüssigkeitsbehältern (31, 32), von denen jeder einzelne entweder mit dem ersten oder sechsten Matrixkanal (24, 29) [das sind die vom Durchflusskanal (30) am weitesten entfernt liegenden Matrixkanäle], benachbart ist,
(iv)Begrenzungen, die mindestens aus zwölf Säulen (23) bestehen und die benachbarten Kanäle (24, 25, 26, 27, 28, 29, 30) voneinander und das erste und sechste Matrixkanal (24, 29) von den zu ihnen benachbarten Flüssigkeitsbehältern (31, 32) trennen,
(v) insgesamt mindestens fünf verscheidenen Zellarten
besteht.

2. Eine Vorrichtung nach Anspruch 1, **dadurch gekennzeichnet, dass** das eine Ende ihres Durchflusskanals (30) über den Anschluss (33) und den Schlauch (39) an die Luftbläschen-Auffangvorrichtung (38), die mit der strömungsregulierenden Pumpe (40) verbunden ist, und das andere Ende über den Anschluss (33) und den Schlauch (34) an das Vorratsbehälter (35) angeschlossen ist, in den die Flüssigkeit hineinfließt und in dem die Krebszellen sowie weitere biologische Moleküle und chemische Stoffe, die sich in die Flüssigkeit gemischt haben, sich sammeln können, um analysiert zu werden.

3. Eine Vorrichtung nach Anspruch 1, **dadurch gekennzeichnet; dass** sie mindestens zwei Randbereiche (22) hat, die scherstellen, dass die verschiedenen Flüssigkeiten in den Flüssigkeitsbehältern (31, 32) unvermischt benutzt werden können.

4. Eine Vorrichtung nach Anspruch 1, **dadurch gekennzeichnet, dass** sie abgerundete Innenkanten (21) hat, die die Bildung von Luftbläschen verringern.

5. Eine Vorrichtung nach Anspruch 1, **dadurch gekennzeichnet, dass** die Flüssigkeiten in den Behältern (31, 32, 35) und im Durchflusskanal (30) die zu testenden Nährmedien, physiologischen Pufferlösungen, biologischen Moleküle, chemischen Stoffe oder eine Kombination aus denselben enthalten.

6. Eine Vorrichtung nach Anspruch 1, **dadurch gekennzeichnet, dass** ihre sämtlichen Kanäle (24, 25, 26, 27, 28, 29, 30) und Flüssigkeitsbehälter (31, 32) jeweils eine Zuflussöffnung, die die Hineingabe der zu testenden Nährmedien, physiologischen Pufferlösungen, biologischen Moleküle, chemischen Stoffe oder einer Kombination aus denselben gewährleistet, und eine Abflussöffnung (3, 4, 5, 6, 7, 8, 9, 10, 11, 12, 13, 14, 15, 16, 17, 18, 19, 20) haben, die während der Befüllung den Austritt der enthaltenen Luft oder der zuvor hineingegebenen Flüssigkeit gewährleistet.

7. Eine Vorrichtung nach Anspruch 1, **dadurch gekennzeichnet, dass** ihre sämtlichen Kanäle (24, 25, 26, 27, 28, 29, 30), Flüssigkeitsbehälter (31, 32) und Säulen (23) die gleiche Höhe haben.

8. Eine Vorrichtung nach Anspruch 1, **dadurch gekennzeichnet, dass** die Länge ihrer Matrixkanäle (24, 25, 26, 27, 28, 29) gleich oder unterschiedlich ist im Verhältnis zur Länge des Durchflusskanals (30).

9. Eine Vorrichtung nach Anspruch 1, **dadurch gekennzeichnet, dass** ihre sämtlichen Kanäle (24, 25, 26 ,27, 28, 29, 30) zueinander parallel sind.

10. Eine Vorrichtung nach Anspruch 1, **dadurch gekennzeichnet, dass** ihre sämtlichen Kanäle (24, 25, 26, 27, 28, 29, 30) jeweils eine Länge zwischen 500 Mikrometer und 20 Zentimeter haben.

11. Eine Vorrichtung nach Anspruch 1, **dadurch gekennzeichnet, dass** die horizontale Schnittfläche einer jeden Säule (23) sechseckig, kreisförmig oder elliptisch ist.

12. Eine Vorrichtung nach Anspruch 1, **dadurch gekennzeichnet, dass** die horizontale Schnittfläche einer jeden Säule (23) zwischen 10 Mikrometer und 1 Millimeter liegt.

13. Eine Vorrichtung nach Anspruch 1, **dadurch gekennzeichnet, dass** das Material der lichtdurchlässigen Oberfläche (1) aus Polydimethylsiloxan oder Polystyrol besteht.

14. Eine Vorrichtung nach Anspruch 1, **dadurch gekennzeichnet, dass** das Material des Gewebes (2) der Vorrichtung aus Polydimethylsiloxan oder Polystyrol besteht.

15. Eine Vorrichtung nach Anspruch 1, **dadurch gekennzeichnet, dass** sie eine Kombination von Krebszellen, Makrophagen, Fibroblasten, Endothelzellen, normalen Brustepithelzellen, Myoepithelzellen, normalen Leberepithelzellen, Hepatozyten, normalen Lungenepithelzellen, normalen Knochenzellen enthält.

16. Eine Vorrichtung nach Anspruch 15, **dadurch gekennzeichnet, dass** es sich bei den verwendeten Zellen um Zelllinien und/oder krebskranke Biopsie-Zellen handelt.

17. Eine Vorrichtung nach Anspruch 1, **dadurch gekennzeichnet, dass** mindestens einer der Matrixkanäle (24, 25, 26, 27, 28, 29) eine 3-D-Matrix mit Krebszellen enthält; die übrigen eine 3-D-Matrix ohne Zellen, eine 3-D-Matrix mit normalen Epithelzellen von gleicher Art wie die Krebszellen, eine 3-D-Matrix mit normalen Epithelzellen von unterschiedlicher Art wie die Krebszellen, eine 3-D-Matrix mit Fibroblasten, eine 3-D-Matrix mit Makrophagen, eine 3-D-Matrix mit Myoepithelzellen, eine 3-D-Matrix mit normalen Leberepithelzellen, eine 3-D-Matrix mit Hepatozyten, eine 3-D-Matrix mit normalen Knochenzellen oder eine Kombination aus denselben enthalten.

18. Eine Vorrichtung nach Anspruch 1, **dadurch gekennzeichnet, dass** die Matrizen in sechs oder weniger Matrixkanälen (24, 25, 26, 27, 28, 29) aus Matrigel, Kollagen, Laminin oder einer Kombination aus denselben bestehen.

19. Eine Vorrichtung nach Anspruch 1, **dadurch gekennzeichnet, dass** der Durchflusskanal (30) die Eigenschaft hat, dass in ihm
(i) die Krebszellen, die mit den Endothelzellen im Durchflusskanal (30) in Berührung kommen,
(ii) die Krebszellen, die die Endothelzellen passieren und in den Durchflusskanal (30) und in die Strömung gelangen,
(iii)die Krebszellen, die von einer Seite des Durchflusskanals (30) in dieselbe hineingelangen und mit den Endothelzellen auf der gegenüberliegenden Seite des Durchflusskanals (30) in Berührung kommen,
mit dem Mikroskop beobachtet werden können.

20. Eine Vorrichtung nach Anspruch 1, **dadurch gekennzeichnet, dass** sie über einen dritten und einen vierten, mit dem Durchflusskanal (30) benachbarten Matrixkanal (26, 27) verfügt, in denen die Bewegungen der Krebszellen in Richtung zu dem Durchflusskanal (30) in der 3-D-Matrix mit oder ohne Zellen und der Austritt der Krebszellen aus dem Durchflusskanal (30) in die 3-D-Matrix mit oder ohne Zellen mit dem Mikroskop beobachtet werden können.

21. Eine Vorrichtung nach Anspruch 1, **dadurch gekennzeichnet, dass** die Matrixkanäle (24, 25, 26, 27, 28, 29) die Eigenschaft haben, dass in ihnen die Existenz und/oder Bildung von neuen Tumoren durch Krebszellen, die den Durchflusskanal (30) passiert und/oder nicht passiert haben, mit dem Mikroskop beobachtet werden können.

22. Eine Vorrichtung nach Anspruch 1, **dadurch gekennzeichnet, dass** es sich bei der Flüssigkeit, die im Durchflusskanal (30) fließt, um das vom Krebspatienten entnommene Blut handelt.

23. Eine Vorrichtung nach Anspruch 1, **dadurch gekennzeichnet, dass** es sich bei der Flüssigkeit, die im Durchflusskanal (30) fließt, um das vom gesunden Menschen entnommene Blut handelt.

24. Eine Methode, mit dem festgestellt wird, ob ein biologisches Molekül, ein chemischer Stoff oder eine Kombination aus denselben die Ausbreitung von Krebs verhindert oder nicht, **dadurch gekennzeichnet, dass**
a. das zu testende biologische Molekül, der chemischer Stoff oder eine Kombination aus denselben in einen oder in mehrere der Flüssigkeitsbehälter (31, 32) und/oder Vorratsbehälter (35) eingegeben wird und mindestens einer der Matrixkanäle (24, 25, 26, 27, 28, 29) eine 3-D-Matrix mit Krebszellen enthält; die übrigen eine 3-D-Matrix ohne Zellen, eine 3-D-Matrix mit normalen Epithelzellen von gleicher Art wie die Krebszellen, eine 3-D-Matrix mit normalen Epithelzellen von unterschiedlicher Art wie die Krebszellen, eine 3-D-Matrix mit Fibroblasten, eine 3-D-Matrix mit Makrophagen, eine 3-D-Matrix mit Myoepithelzellen, eine 3-D-Matrix mit normalen Leberepithelzellen, eine 3-D-Matrix mit Hepatozyten, eine 3-D-Matrix mit normalen Knochenzellen oder eine Kombination aus denselben enthalten und
b. es sich um die Feststellung handelt, ob in einem Matrixkanal - abgesehen von dem Matrixkanal, in den ursprünglich Krebszellen platziert worden sind - neue Tumoren entstehen oder nicht, **dadurch gekennzeichnet, dass**, falls in einem Matrixkanal - abgesehen von dem Matrixkanal, in den ursprünglich Krebszellen platziert worden sind - keine neuen Tumoren entstehen sollten, dies darauf hinweist, dass das getestete biologische Molekül, der chemischer Stoff oder eine Kombination aus denselben - verglichen mit einer adäquaten Kontrolle eines biologischen Moleküls oder chemischen Stoffes - gegen die Ausbreitung von Krebs verwendet werden kann.

25. Eine Methode, mit dem festgestellt wird, welches Gewebe krebskranke Epithelzellen für ihre Ausbreitung anzielen, **dadurch gekennzeichnet, dass**
a. in der Vorrichtung nach Anspruch 1 mindestens einer der Matrixkanäle (24, 25, 26, 27, 28, 29) eine zu testende 3-D-Matrix mit Krebszellen enthält; die übrigen eine 3-D-Matrix ohne Zellen, eine 3-D-Matrix mit normalen Epithelzellen von gleicher Art wie die Krebszellen, eine 3-D-Matrix mit normalen Epithelzellen von unterschiedlicher Art wie die Krebszellen, eine 3-D-Matrix mit Fibroblasten, eine 3-D-Matrix mit Makrophagen, eine 3-D-Matrix mit Myoepithelzellen, eine 3-D-Matrix mit normalen Leberepithelzellen, eine 3-D-Matrix mit Hepatozyten, eine 3-D-Matrix mit normalen Knochenzellen oder eine Kombination aus denselben enthalten.
b. es sich um die Feststellung der Matrixkanäle handelt, in denen ein neuer Tumor entsteht oder sich befindet, **dadurch gekennzeichnet, dass**,
wenn ein neuer Tumor in dem Matrixkanal entsteht, in dem sich normale Lungenepithelzellen befinden, dies darauf hinweist, dass die getesteten Krebszellen sich in der Lunge ausbreiten werden; wenn ein neuer Tumor in dem Matrixkanal entsteht, in dem sich normale Knochenzellen befinden, dies darauf hinweist, dass die getesteten Krebszellen sich in den Knochen ausbreiten werden;
wenn ein neuer Tumor in dem Matrixkanal entsteht, in dem sich normale Leberzellen befinden, dies darauf hinweist, dass die getesteten Krebszellen sich in der Leber ausbreiten werden.

## Revendications

1. L'invention est un dispositif microfluidique, **caractérisée en ce que** la surface perméable à la lumière (1) sur laquelle se trouve le tissu de l'appareil (2) inclue
(i) au moins un canal de flux (30) comprenant des cellules endothéliales,
(ii) au moins trois canaux adjacents, contenant une matrice 3D (trois dimensions) avec ou sans cellules, adjacente à chaque bord longitudinal du canal de flux, au total au moins six canaux matriciels (24, 25, 26, 27, 28, 29) contenant une matrice 3D (trois dimensions) avec ou sans cellules,
(iii) au moins deux récipients de fluide (31, 32), dont chacun est adjacent au premier et sixième canaux matriciels (24, 29) canaux les plus éloignés du canal de flux (30),
(iv) les limites composées par au moins douze colonnes (23) séparant les uns des autres chaque canaux voisins (24, 25, 26 ,27, 28, 29, 30) et les réservoirs de fluides (31, 32) adjacents aux premier et sixième canaux matriciels (24, 29),
(v) au total au moins cinq types de cellules diférentes.

2. Appareil selon la revendication 1 **caractérisé en ce que** ledit canal de flux (30) dont l'une des extrémités est raccordé (33) par un tuyau (39) régulant le flux à une pompe (40) sur laquelle est liée une trappe (38) de bulle d'air et l'autre extremité (33) reliée par un tuyau (34) dans lequel passe le flux et où se mélange les cellules cancéreuses et les autres molécules biologiques et produits chimiques collectés dans le réservoir de stockage (35) à des fins d'analyses.

3. Appareil selon la revendication 1 **caractérisé en ce que** dans lesdits réservoirs de stockage (31, 32) permettant aux différents fluides d'être utilisés sans se mélanger, qu'il y ait au moins deux zones (22) voisines.

4. Appareil selon la revendication 1 **caractérisé en ce qu'**il y ait à l'intérieur des coins arrondis (21) permettant de réduire la formation de bulles d'air.

5. Appareil selon la revendication 1 **caractérisé en ce qu'**il comprenne les fluides des réservoirs (31, 32, 35) et du canal de flux (30), les milieux de cultures dans lesquels sont effectués les tests, les solutions de tampon physiologique, les molécules biologiques, les produits chimiques ou bien les divers combinaisons de ces derniers.

6. Appareil selon la revendication 1 **caractérisé en ce qu'**il y ait dans tous les canaux (24, 25, 26, 27, 28, 29, 30) et les réservoirs de fluides (31, 32) une entrée permettant le chargement des milieux de culture dans lesquels sont effectués les tests, les solutions de tampon physiologique, les molécules biologiques ou bien les produits chimiques, les matrices avec cellules, les matrices sans cellules ou bien les divers combinaison de ces derniers ainsi qu'une sortie permettant à l'air ou aux fluides introduits lors de l'installation de sortir (3, 4, 5, 6, 7, 8, 9, 10, 11, 12, 13, 14, 15, 16, 17, 18, 19, 20).

7. Appareil selon la revendication 1 **caractérisé en ce que**, lesdits canaux (24, 25, 26 ,27, 28, 29, 30), lesdits réservoirs (31, 32) et lesdites colonnes (23) soient à la même hauteur.

8. Appareil selon la revendication 1 **caractérisé en ce que**, lesdits canaux matriciels (24, 25, 26, 27, 28, 29) soient avec ledit canal de flux (30) à des longueurs identiques ou différentes.

9. Appareil selon la revendication 1 **caractérisé en ce que**, tous lesdits canaux (24, 25, 26, 27, 28, 29, 30) soient parallèles les uns aux autres.

10. Appareil selon la revendication 1 **caractérisé en ce que**, tous lesdits canaux (24, 25, 26, 27, 28, 29, 30) possèdent chacun une longueur comprise entre 500 micromètre et 20 centimètre.

11. Appareil selon la revendication 1 **caractérisé en ce que**, lesdites colonnes (23) soient chacune, des coupes horizontales hexagonales, circulaires ou bien éliptiques.

12. Appareil selon la revendication 1 **caractérisé en ce que**, lesdites colonnes (23) aient chacune des coupes horizontales comprises entre 10 micromètres et 1 milimètre.

13. Appareil selon la revendication 1 **caractérisé en ce que**, ladite surface perméable à la lumière (1) soit constituée de matériaux en polydiméthylsiloxane ou bien en polystyrène.

14. Appareil selon la revendication 1 **caractérisé en ce que**, ledit tissu de l'appareil (2) soit constitué de matériaux en polydiméthylsiloxane ou bien en polystyrène.

15. Appareil selon la revendication 1 **caractérisé en ce qu'**il comprenne une combinaison des cellules cancéreuses, des macrophages, des fibroblastes, des cellules endothéliales, des cellules épithéliales du sein, des cellules myoépithéliales, des cellules épithéliales normales du foie, des hépatocytes, des cellules épithéliales pulmonaires normales, des cellules osseuses normales.

16. Appareil selon la revendication 1 **caractérisé en ce que**, lesdites cellules utilisées soient une lignée cellulaire et/ou des cellules cancéreuses de biopsie.

17. Appareil selon la revendication 1 **caractérisé, en ce que** parmi lesdits canaux matricels (24, 25, 26, 27, 28, 29) au moins un contienne une matrice de cellules cancéreuses 3D, et les autres des matrices 3D sans cellules, des matrices 3D avec le même type de cellules cancéreuses épithéliales normales, des matrices 3D de cellules épithéliales normales de différents types que les cellules cancéreuses, des matrices de fibroblastes 3D, des matrices macrophages 3D, des matrices de cellules myoépithéliales 3D, des matrices de cellules épithéliales de foie normal, des matrices d'hépatocytes, des matrices de cellules osseuses normales 3D, ou bien une combinaison de ces derniers.

18. Appareil selon la revendication 1 **caractérisé en ce que**, six ou beaucoup moins des matrices contenues dans lesdits canaux matriciels (24, 25, 26, 27, 28, 29) soient constituées de matrigel, collagène et laminine ou bien une combinaison de ces dernières.

19. Appareil selon la revendication 1 **caractérisé en ce que**; ledit canal de flux (30) soit un canal
(i) dans lequel (30) les cellules cancéreuses entrent en contact avec les cellules endothéliales,
(ii) dans lequel les cellules cancéreues dépassent les cellules endothéliales et entrent dans le canal de flux (30) en suivant l'ecoulement,
(iii) dans lequel il est possible d'observer au microscope les cellules cancéreuses lesquelles entrent d'un côté du canal de flux (30) pour être en contact avec les cellules endothéliales de l'autre côté du canal de flux (30).

20. Appareil selon la revendication 1 **caractérisé en ce que**; adjacent au canal de flux (30), les cellules cancéreuses, contenues dans les matrices avec ou sans cellules 3D, se déplaçant vers le canal de flux (30) et lesdites cellules cancéreuses allant du canal de flux à la sortie interne de la matrice 3D avec ou sans cellules sont observables au microscope par les troisième et quatrième canaux matriciels (26, 27).

21. Appareil selon la revendication 1 **caractérisé en ce que**; le canal de flux (30) desdits canaux matriciels (24, 25, 26, 27, 28, 29) soit traversé et/ou non par des cellules cancéreuses ainsi la formation et/ou la présence de nouvelle tumeur peut être observer au microscope par l'existence de ces canaux.

22. Appareil selon la revendication 1 **caractérisé en ce que**; le fluide coulant dans ledit canal de flux (30) soit le sang pris du malade cancéreux.

23. Appareil selon la revendication 1 **caractérisé en ce que**; le fluide coulant dans ledit canal de flux (30) soit le sang pris d'un tiers en bonne santé.

24. Une molécule biologique, chimique ou bien la combinaison de ces derniers pouvant être un procédé pour déterminer si la propagation du cancer doit être empêcher ou non, laquelle se **caractérise en ce que**;
a. Les molécules biologique, chimique ou bien la combinaison de ces derniers devant être testées, sont à ajouter à l'une ou plusieurs réservoirs de fluides (31, 32) de l'appareil selon la revendication 1 et/ou au réservoir de stockage (35) etdes canaux matriciels (24, 25, 26, 27, 28, 29) au moins un comprend une matrice de cellules cancéreuses 3D, les autres comprennent une matrice sans cellules 3D, une matrice de cellules épithéliales normales de même type de cancer 3D, une matrice de cellules épithéliales normales de différentes types de cancer 3D, une matrice fibroblaste 3D, une matrice macrophage 3D, matrice de cellules myoépithéliales 3D, matrice de cellules épithéliales de foie normal 3D, une matrice hépatocytes 3D, une matrice de cellules osseuses 3D ou bien une combinaison de ces derniers et
b. Outre le canal matriciel auquel ont été ajouté au début des cellules cancéreuses , il est permis de déterminer la formation ou non de nouvelle tumeur dans un canal matriciel ce qui se **caractérise en ce que**;
En dehors du canal matriciel de départ où les cellules cancéreuses ont été ajoutées, si de nouvelles tumeurs ont été formées dans un canal matriciel, que le test de la molécule biologique, chimique ou bien la combinaison de celles-ci est comparé avec le contrôle adapté d'une molécule biologique ou bien chimique, alors il informe de son utilisation possible contre la propagation du cancer.

25. Etant le procédé qui précise quel tissu va être pris pour cible pour propager des cellules épithéliales cancéreuses, procédé qui se **caractérise en ce que**;
a. Dans l'appareil de la revendication 1 au moins un des canaux matricels (24, 25, 26, 27, 28, 29) devant être testé doit comporter une matrice de cellule cancéreuse 3D, les autres une matrice sans cellule 3D, une matrice de cellules épithéliales normales de même type de cancer 3D, une matrice de cellules épithéliales normales de différentes types de cancer 3D, une matrice fibroblaste 3D, une matrice macrophage 3D, matrice de cellules myoépithéliales 3D, matrice de cellules épithéliales, de foie normal 3D, une matrice hépatocytes 3D, une matrice de cellules osseuses 3D ou bien la combinaison de ces derniers et
b. La détermination de canaux matriciels contenant à l'intérieur la formation ou l'existance d'une nouvelle tumeur se **caractérise en ce que** ;
Si une nouvelle tumeur se forme dans le canal matriciel dans lequel se trouve des cellules épithéliales normales des poumons , alors cela démontre que ces cellules cancéreuses testées vont se propager aux poumons, si une nouvelle tumeur se forme dans le canal matriciel dans lequel se trouve les cellules, osseuses normales, alors cela démontre que ces cellules cancéreuses testées vont se propager aux os, si une nouvelle tumeur se forme dans, le canal matriciel dans lequel se trouve des cellules du foie normales, alors cela démontre que ces cellules cancéreuses testées vont se propager au foie.
